# EUROPEAN PATENT SPECIFICATION

(11) **EP 3 406 214 B1**
(45) Date of publication and mention of the grant of the patent: **05.06.2024**
(21) Application number: 16886290.2
(22) Date of filing: 20.01.2016
(51) Int. Cl.: A61B 18/00, A61B 17/32

(54) **MEDICAL APPARATUS, MEDICAL APPARATUS SYSTEM**
MEDIZINISCHE VORRICHTUNG, SYSTEM MIT MEDIZINISCHER VORRICHTUNG
APPAREIL MÉDICAL, SYSTÈME D'APPAREIL MÉDICAL

(43) Date of publication of application: 28.11.2018
(73) Proprietor: OLYMPUS CORPORATION, Hachioji-shi Tokyo 192-8507 (JP)
(72) Inventor: AKAGANE, Tsunetaka, Tokyo 192-8507 (JP)
(74) Representative: Gunzelmann, Rainer
(86) International application number: PCT/JP2016/051536
(87) International publication number: WO 2017/126048

(56) References cited:
- JP-A- 2002 085 420
- JP-B2- 5 635 720
- US-A1- 2009 270 891
- US-A1- 2010 168 741
- US-A1- 2014 324 084
- US-A1- 2014 378 971

## Description

### FIELD

The present invention relates to vibration transmission member and a medical device for treating body tissue with energy of ultrasonic vibration, etc.

### BACKGROUND

There is an ultrasonic treatment apparatus that uses ultrasonic waves to perform treatment such as incising, excising, and coagulating body tissue, as disclosed in Patent Literature 1. The ultrasonic treatment apparatus includes an ultrasonic probe to which ultrasonic vibration is transmitted, and a jaw that opens and closes relative to the ultrasonic probe. An asymmetrical portion is formed in the ultrasonic probe, so that the ultrasonic probe is shaped to include an arc-shaped bend portion. The ultrasonic treatment apparatus can perform incision, etc., of the body tissue by transmitting ultrasonic vibration to the ultrasonic probe while grasping the body tissue between the ultrasonic probe and the jaw.

US 2009/270891 concerns methods and devices that provide reduced transverse motion in a curved ultrasonic blade and/or ultrasonic surgical instrument with functional asymmetries. An ultrasonic blade includes a curved functional portion of an ultrasonic blade, wherein the center of mass of the curved functional portion lies on the mid-line of a waveguide delivering ultrasonic energy to the blade.

US 2014/378971 A1 discloses a treatment device for adjusting the vibration velocity distribution along a longitudinal direction of a probe of the device. To this end, the probe is provided with a proximal end side that has a larger thickness than a distal end side of the probe.

### CITATION LIST

### PATENT LITERATURE

Patent Literature 1: Jpn. PCT National Publication No. 2008-264565

### SUMMARY

### TECHNICAL PROBLEM

In the medical equipment using an ultrasonic wave, there was a request to the medical equipment which has the stability of ultrasonic vibration and can realize efficient ultrasonic vibration.

### SOLUTION TO PROBLEM

The present invention concerns a vibration transmission member according to claim 1 and a medical device according to claim 11. Further aspects of the present invention are defined in the dependent claims.

A medical device to which ultrasonic vibration for resonating a vibration transmission member is transmitted from a transducer that generates the ultrasonic vibration, the medical device comprising: the vibration transmission member comprising a distal end portion, a proximal end portion provided closer to the transducer, and an intermediate portion provided between the distal end portion and the proximal end portion; a first cross-sectional area reduction portion, which is provided to the distal end portion, and makes a cross-sectional area of the distal end portion concerning a plane intersecting with a central axis of the vibration transmission member smaller than a cross-sectional area of the intermediate portion concerning a plane intersecting with the central axis; and a second cross-sectional area reduction portion, at which a node position of the ultrasonic vibration nearest to the distal end portion is located while the vibration transmission member is resonating, and which is provided opposite to the first cross-sectional area reduction portion with respect to the central axis, the second cross-sectional area reduction portion making a cross-sectional area concerning a plane intersecting with the central axis of a half portion of the vibration transmission member located opposite to the first cross-sectional area reduction portion smaller than a cross-sectional area concerning a plane intersecting with the central axis of a half portion of the vibration transmission member located closer to the first cross-sectional area reduction portion.

### ADVANTAGEOUS EFFECTS OF INVENTION

According to the configuration described above, a medical device that is capable of implementing efficient ultrasonic vibration can be provided.

### BRIEF DESCRIPTION OF THE DRAWING(S)

FIG. 1 is a schematic diagram showing an overall configuration of a medical device according to a first embodiment.
FIG. 2 is a perspective view showing a jaw and a distal end portion of a vibration transmission member of a handpiece of the medical device shown in FIG. 1.
FIG. 3 is a cross-sectional view of a vibrator unit of the medical device shown in FIG. 1.
FIG. 4 is an enlarged perspective view of a distal end portion and an intermediate portion of a probe of the medical device shown in FIG. 1.
FIG. 5 is a plan view of the distal end portion and the intermediate portion of the probe shown in FIG. 4, which are viewed from a treatment face side.
FIG. 6 is a side view of the distal end portion, the intermediate portion, and a proximal end portion of the probe shown in FIG. 4, which are viewed from a bending direction side of a bend portion.
FIG. 7 is a cross-sectional view of the probe shown in FIG. 6 taken along line F7-F7.

### DETAILED DESCRIPTION

### [First Embodiment]

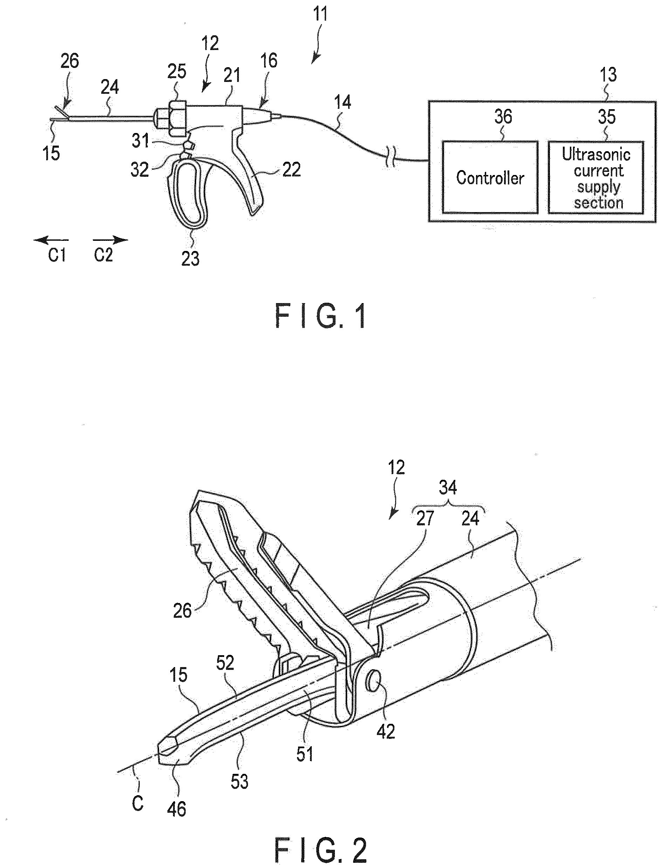

An embodiment of an energy treatment apparatus of the present invention will be described with reference to FIGS. 1 to 7.

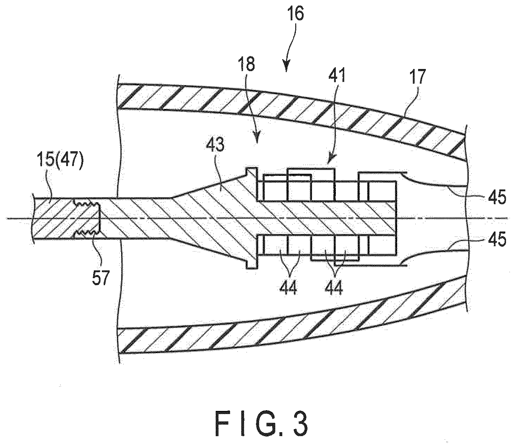

As illustrated in FIGS. 1 and 3, an energy treatment apparatus 11 (medical device system) includes: a handpiece 12 (medical device); a power source unit 13; a cable 14 that connects the handpiece 12 and the power source unit 13; and a vibrator unit 16 (transducer) that supplies ultrasonic vibration (ultrasonic energy) for resonating a probe 15 of the handpiece 12.

The vibrator unit 16 includes a case 17 that is detachable from the housing 21 of the handpiece 12 (medical device), and a vibration generator 18 that is stored in the case 17. Also, the medical device system disclosed by the present embodiment includes, for example, the handpiece 12 and the vibrator unit 16 (transducer). In the present embodiment, one of two directions parallel to the central axis C (longitudinal axis) of the probe 15 is referred to as a distal direction C1, and a direction opposite to the distal direction C1 is referred to as a proximal direction C2.

As illustrated in FIGS. 1 to 3, and 6, the handpiece 12 (medical device) includes: the housing 21 forming a part of an outer shell; a grip portion 22 protruding in a rod shape from the housing 21; a movable handle 23 attached to the housing 21 so as to be rotatable with respect to the grip portion 22; the probe 15 (vibration transmission member) having a rod shape and connected (fixed) to the vibration generator 18; a cylindrical sheath 24 attached to the housing 21 so as to cover a periphery of the probe 15; a knob 25 fixed to the sheath 24; a jaw 26 provided to be rotatable with respect to the probe 15 and the sheath 24; a cylindrical movable pipe 27 that is provided inside the sheath 24 and is advanced and retreated when the jaw 26 is opened and closed; a first button 31 and a second button 32 that are provided to the housing 21 and switch on and off the ultrasonic vibration output from the vibration generator 18; and an annular seal member 33 (elastic member) interposed between the probe 15 and the movable pipe 27. The seal member 33 is made of, for example, a rubber material. A sheath-shaped section 34 is formed of the sheath 24 and the movable pipe 27.

As illustrated in FIG. 1, the power source unit 13 includes an ultrasonic current supply section 35 (ultrasonic energy supply section) and a controller 36 that controls the ultrasonic current supply section 35. The controller 36 can control power supply from the ultrasonic current supply section 35. When the first button 31 or the second button 32 is pushed by a doctor, the controller 36 supplies power (alternating-current power) from the ultrasonic current supply section 35 to the vibration generator 18. The first button 31 located on the probe 15 side, for example, outputs ultrasonic energy with reduced amplitude of ultrasonic vibration, to handle a seal-mode treatment (function) to coagulate body tissue and stanch blood. The second button 32 located on the movable handle side, for example, outputs ultrasonic energy with increased amplitude of ultrasonic vibration, to mainly handle a cut-mode treatment (function) to incise body tissue.

In the present embodiment, ultrasonic energy is used for the treatment; however, treatment energy is not limited to only ultrasonic energy. As the treatment energy, high-frequency current energy or thermal energy may be used in combination with ultrasonic energy. Namely, treatment energy combining ultrasonic energy and high-frequency current energy may be applied to body tissue from the probe 15, and treatment energy combining ultrasonic energy and thermal energy may be applied to body tissue from the probe 15.

The movable handle 23 is attached to be movable (rotatable) relative to the housing 21. By bringing the movable handle 23 closer to or away from the grip portion 22, the doctor advances and retreats the movable pipe 27 inside the sheath 24, thereby allowing the jaw 26 to be opened and closed.

The sheath 24 is formed in a cylindrical shape by a metallic material, etc., and protects the probe 15 located thereinside. The proximal direction C2 side of the sheath 24 is attached to the housing 21 to be rotatable with respect to the housing 21. The knob 25 is fixed to the sheath 24, and is attached to be rotatable with respect to the housing 21. By rotating the knob 25 with respect to the housing 21, the sheath 24, the probe 15, an ultrasonic vibrator 41, and the jaw 26 can be integrally rotated about the longitudinal axis C (central axis).

The jaw 26 is supported by a support pin 42 provided to a distal end portion of the sheath 24. The jaw 26 is rotatable about the support pin 42 between a contact position where the jaw 26 is in contact with the probe 15 or close to the probe 15 to face the probe 15, and a spaced position where the jaw 26 is spaced apart from the probe 15.

As shown in FIG. 3, the vibration generator 18 includes the ultrasonic vibrator 41 and a horn member 43. The ultrasonic vibrator 41 includes a plurality of (e.g., four) piezoelectric elements 44 for changing a current into ultrasonic vibration. The ultrasonic vibrator 41 is connected to one end of an electrical wiring 45. The electrical wiring 45 extends inside the cable 14, so that the other end of the electrical wiring 45 is connected to the ultrasonic current supply section 35 of the power source unit 13. When power is supplied from the ultrasonic current supply section 35 to the ultrasonic vibrator 41 via the electrical wiring 45, ultrasonic vibration (vibration in the central axis C direction, namely, longitudinal vibration) is generated in the ultrasonic vibrator 41. The vibration generator 18 can resonate the probe 15 by transmitting this ultrasonic vibration to the probe 15 side as well. A frequency of the ultrasonic vibration generated by the vibration generator 18 is, for example, 47 kHz, and is 46 kHz or more and 48 kHz or less in an example.

As shown in FIG. 3, the ultrasonic vibrator 41 is attached to the horn member 43. The horn member 43 is made of a metallic material. The horn member 43 includes an approximately cone-shaped cross-section transition portion, a cross-sectional area of which decreases in the distal direction C1 of the probe 15. The amplitude of the ultrasonic vibration generated in the ultrasonic vibrator 41 is increased in the cross-section transition portion.

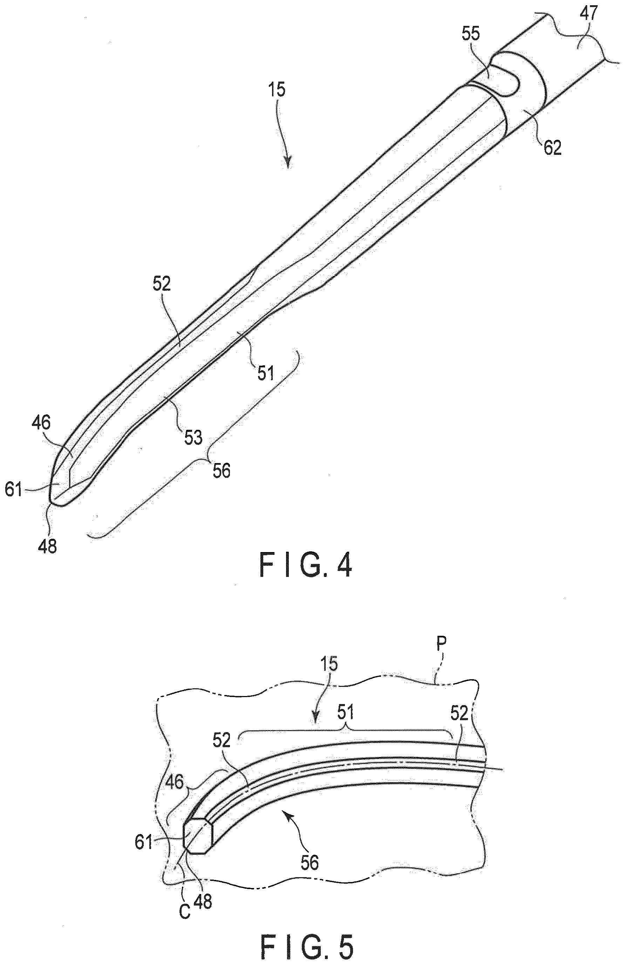

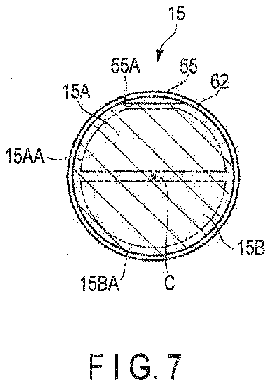

The probe 15 (vibration transmission member) is formed of, for example, a biocompatible metallic material (e.g., a titanium alloy, etc.). As illustrated in FIGS. 4 to 6, the probe 15 includes: a distal end portion 46 located on the distal direction side; a proximal end portion 47 located on the proximal direction side opposite to the distal end portion 46; a distal end face 48 provided at the farthest end of the distal direction side of the distal end portion 46; an intermediate portion 51 located between the distal end portion 46 and the proximal end portion 47; a treatment face 52 provided astride the distal end portion 46 and the intermediate portion 51; a back face 53 (opposite face) located opposite to the treatment face 52; an inclined portion 54 (first cross-sectional area reduction portion) provided to the distal end portion 46 and inclined with respect to the central axis C; a groove 55 (second cross-sectional area reduction portion) provided apart from the treatment face 52; and an annular second groove 62 (depressed portion) provided at a position overlapping with the groove 55. Also, as illustrated in FIG. 7, the probe 15 includes two portions extending along the central axis C, which is interposed between the two portions, namely, includes a half portion 15A on the treatment face 52 side and a half portion 15B on the back face 53 side.

As illustrated in FIG. 5, a portion of the distal end portion 46 and a portion of the intermediate portion 51 that correspond to the treatment face 52 form a bend portion 56 that is bent as it is closer to the distal direction side of the probe 15, e.g., bent leftward. The bending direction of the bend portion 56 may be discretionarily set, and may be suitably set according to the type of treatment. As illustrated in FIGS. 1 and 2, a periphery of the probe 15 on the proximal direction C2 side is supported by the housing 21. Also, as illustrated in FIG. 3, the proximal side of the probe 15 is fixed to the horn member 43 so as to be pressed against the horn member 43 at a predetermined pressure via a screw 57. The vicinity of an antinode position of ultrasonic vibration is located at the distal end face 48 and the distal end portion 46 in a state where the probe 15 performs ultrasonic vibration resonance.

The treatment face 52 forms a portion that is flat and is brought into contact with body tissue at the time of treatment. The treatment face 52 is provided opposite to the inclined portion 54 (first cross-sectional area reduction portion) with respect to the central axis C.

The inclined portion 54 is provided on the back face 53 side. The inclined portion 54 makes a cross-sectional area of the distal end portion 46 concerning a plane intersecting with the central axis C of the probe 15 smaller than a cross-sectional area of the intermediate portion 51 concerning a plane intersecting with the central axis C. More specifically, the inclined portion 54 is formed so that the cross-sectional area of the distal end portion 46 concerning the plane intersecting with the central axis C is gradually decreased as the inclined portion 54 is closer to the distal end face 48 located at the farthest end of the distal side of the distal end portion 46. A second inclined portion 61 that is inclined with respect to the central axis C is also provided on the treatment face 52 side in the distal end portion 46 of the probe 15. Therefore, the distal end portion 46 of the probe 15 has a tapered shape including the inclined portion 54 and the second inclined portion 61 on both sides.

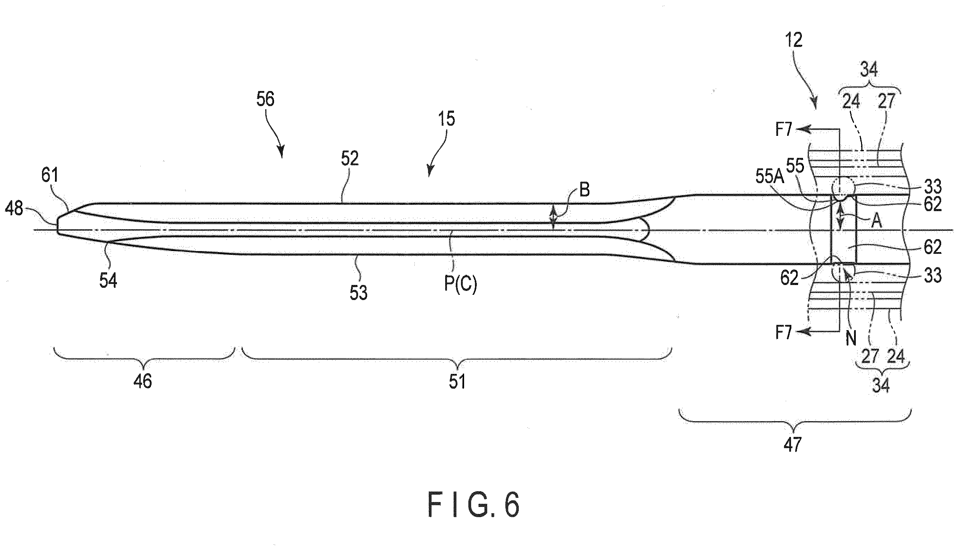

The groove 55 (second cross-sectional area reduction portion) is provided on the treatment face 52 side of the proximal end portion 47, but is provided apart from the treatment face 52 (i.e., closer to the proximal direction C2 side than the treatment face 52) as viewed in the central axis C direction. A node position N of the aforementioned ultrasonic vibration nearest to the distal end portion 46 is located at the groove 55 in a state where the probe 15 performs ultrasonic vibration resonance. The groove 55 is provided opposite to the inclined portion 54 (first cross-sectional area reduction portion) with respect to the central axis C. More specifically, the groove 55 is provided opposite to the inclined portion 54 at an angle of approximately 180 degrees around the central axis C (e.g., at a position at an angle of 175 degrees or 185 degrees in relation to the inclined portion 54 around the central axis C). If a plane P passing along the central axis C and including the entire bend portion 56 (or a plane P approximately parallel to the bending direction of the bend portion 56) is considered, as shown in FIG. 5, the groove 55 (second cross-sectional area reduction portion) is provided opposite to the inclined portion 54 with respect to the plane P, as shown in FIG. 6.

As illustrated in FIG. 6, the groove 55 (second cross-sectional area reduction portion) has an irregular cross-sectional shape (e.g., a polygonal shape such as triangle or quadrilateral, a semicircular shape, a semioval shape, a parabolic shape, a curved surface shape, etc., excluding a perfect circular shape), when cut along a plane passing along the central axis C. The groove 55 more preferably has an approximately semicircular cross-sectional shape when cut along a plane passing along the central axis C. As illustrated in FIG. 7, the groove 55 makes a cross-sectional area 15AA concerning a plane intersecting with the central axis C of the half portion 15A of the probe 15 located opposite to the inclined portion 54 smaller than a cross-sectional area 15BA concerning a plane intersecting with the central axis C of the half portion 15B of the probe 15 located on the inclined portion 54 side. As illustrated in FIGS. 4 and 6, the groove 55 extends in a direction intersecting with the central axis C (more specifically, an orthogonal direction), but is actually provided at a position (twist position) apart from the central axis C at a predetermined distance without intersecting with the central axis C. The groove 55 is formed by, for example, cutting processing using a milling machine, but may also be formed by other processing methods. The groove 55 includes a bottom portion 55A. A distance A from the bottom portion 55A to the central axis C is larger than a distance B from the treatment face 52 to the central axis C. The groove 55 has a shape different from that of the inclined portion 54, and has a shape asymmetrical to that of the inclined portion 54 with respect to the central axis C.

The second groove 62 (depressed portion) is provided at a position overlapping with the groove 55 (second cross-sectional area reduction portion). Therefore, the groove 55 (first groove) is provided to be further dented from the second groove 62. The second groove 62 is provided to be dented in an annular shape with respect to the probe 15. A part of the annular seal member 33 is disposed inside the second groove 62. Therefore, the groove 55 is covered with the seal member 33.

Next, an action performed with the energy treatment apparatus 11 of the present embodiment will be described. For example, a doctor can hold the handpiece 12 of the energy treatment apparatus 11 with the right hand (or with the left hand, if desired). By pulling the movable handle 23 toward the grip portion 22 side with the middle finger, the ring finger, and the little finger of the right hand (or the left hand), the doctor can rotate the jaw 26 and bring the jaw 26 into contact with the treatment face 52 of the probe 15 or make the jaw 26 face the treatment face 52 of the probe 15 with a slight gap therebetween. When the body tissue is present between the jaw 26 and the treatment face 52, the body tissue can be held between the treatment face 52 and the jaw 26 like forceps. By returning the movable handle 23 to its original position, the jaw 26 is spaced from the treatment face 52, so that the body tissue can be released. Furthermore, when the doctor pushes the first button 31 or the second button 32 with the forefinger of the right hand (or the left hand), the controller 36 controls the ultrasonic current supply section 35 and turns on the output of the ultrasonic vibration from the ultrasonic vibrator 41, so that the ultrasonic vibration can be applied to the body tissue from the probe 15. By releasing the push of the first button 31 or the second button 32, the doctor can turn off the output of the ultrasonic vibration from the ultrasonic vibrator 41.

An operation of the energy treatment apparatus 11 of the present embodiment will be described. First, the impedance of the ultrasonic vibration was measured for an example (comparative example) in which the groove 55 (second cross-sectional area reduction portion) was not provided to the probe 15. A probe of the comparative example (not shown in the drawings) was connected to the vibrator unit 16 (transducer) to drive the vibrator unit 16, and the probe 15 and the vibrator unit 16 were subjected to ultrasonic vibration (were resonated) at, for example, 47 kHz. When the impedance of the ultrasonic vibration that can be considered as resistance (vibration loss) for maintaining the resonance of the ultrasonic vibration was measured in this state, the impedance was 275 Ω.

Next, in place of the probe of the comparative example, the probe 15 of the present embodiment was connected to the vibrator unit 16 (transducer). When the vibrator unit 16 was driven, and the probe 15 and the vibrator unit 16 were subjected to ultrasonic vibration (were resonated) at, for example, 47 kHz in this state, the impedance of the ultrasonic vibration decreased to 148 Ω. Therefore, the energy treatment apparatus 11 of the present embodiment allowed decrease of the impedance of the ultrasonic vibration by about 46%, as compared to the comparative example.

According to the first embodiment, the medical device is a medical device to which ultrasonic vibration is transmitted from a transducer that generates the ultrasonic vibration for resonating a vibration transmission member. The medical device includes: the vibration transmission member, which includes the distal end portion 46, the proximal end portion 47 provided on the transducer side, and the intermediate portion 51 provided between the distal end portion 46 and the proximal end portion 47; the first cross-sectional area reduction portion, which is provided to the distal end portion 46, and makes a cross-sectional area of the distal end portion 46 concerning a plane intersecting with the central axis C of the vibration transmission member smaller than a cross-sectional area of the intermediate portion 51 concerning a plane intersecting with the central axis C; and the second cross-sectional area reduction portion, at which the node position N of the ultrasonic vibration nearest to the distal end portion is located while the vibration transmission member is resonating, and which is provided opposite to the first cross-sectional area reduction portion with respect to the central axis C, the second cross-sectional area reduction portion making a cross-sectional area concerning a plane intersecting with the central axis of the half portion 15A of the vibration transmission member located opposite to the first cross-sectional area reduction portion smaller than a cross-sectional area concerning a plane intersecting with the central axis C of the half portion 15B of the vibration transmission member located on the first cross-sectional area reduction portion side.

With the above-described medical device, it is possible to, for example, incise, excise, and coagulate the body tissue using the longitudinal vibration that causes the vibration transmission member to vibrate in a direction along the central axis C. According to the above configuration, an abnormal vibration other than the originally-planned longitudinal vibration (a flexural vibration generated in a direction intersecting with the central axis C, a torsional vibration generated in a torsional direction around the central axis C, a harmonic (second harmonic, third harmonic, etc.) having a frequency that is an integral multiple of the resonant frequency, etc.) is generated in the vibration transmission member by the first cross-sectional area reduction portion. However, by providing the second cross-sectional area reduction portion, it is possible to generate an abnormal vibration (in particular, an abnormal vibration of a flexural vibration generated in a direction intersecting with the central axis C) that is in an opposite phase to the aforementioned abnormal vibration. Therefore, the abnormal vibration generated due to the first cross-sectional area reduction portion is set off (canceled) by the abnormal vibration generated by the second cross-sectional area reduction portion. In particular, when a direction from the treatment face 52 to the back face 53 is defined as a thickness direction, a flexural vibration in the thickness direction generated due to the first cross-sectional area reduction portion is set off by the abnormal vibration generated due to the second cross-sectional area reduction portion. In this case, it is understood that because stress is higher in the vicinity of the node position N of the ultrasonic vibration than in another part (e.g., antinode position), providing the second cross-sectional area reduction portion in this position can achieve an especially high cancellation effect.

Thereby, it is possible to reduce the impedance of the ultrasonic vibration, which is a measure of vibration loss, and it is also possible to ensure the stability of the ultrasonic vibration and improve the efficiency of the ultrasonic vibration. Therefore, it is possible to save power of the medical device, and also possible to reduce an amount of heat generated by vibration loss. In addition, it is possible to reduce repeated bending (flexure) generated in the vibration transmission member by abnormal vibration, and possible to extend the life of the medical device. Furthermore, it is possible to reduce an amount of bubbles generated due to abnormal vibration when the vibration transmission member is used in a liquid.

The second cross-sectional area reduction portion is provided opposite to the first cross-sectional area reduction portion at an angle of 180 degrees around the central axis C. According to this configuration, it is possible, by the second cross-sectional area reduction portion, to most efficiently generate the opposite-phase abnormal vibration for setting off the abnormal vibration generated due to the first cross-sectional area reduction portion, and also possible to save power of the medical device, reduce an amount of heat generated, extend the life, and reduce an amount of bubbles generated.

The second cross-sectional area reduction portion forms the groove 55 that extends in a direction intersecting with the central axis C. According to this configuration, it is possible to simplify the shape of the second cross-sectional area reduction portion. Thereby, it is possible to reduce the costs of processing the vibration transmission member, and also possible to minimize the reduction of the stiffness of the vibration transmission member.

The vibration transmission member includes the treatment face 52 provided opposite to the first cross-sectional area reduction portion with respect to the central axis, and a distance from the bottom portion 55A of the groove 55 formed by the second cross-sectional area reduction portion to the central axis C is larger than a distance from the treatment face 52 to the central axis C. According to this configuration, it is possible to reduce the depth of the groove 55, and to minimize the decrease of the stiffness occurring in the vibration transmission member.

The medical device includes the cylindrical sheath 24 that covers the vibration transmission member, and the annular elastic member interposed between the sheath 24 and the vibration transmission member. The annular depressed portion is provided to the vibration transmission member at a position overlapping with the second cross-sectional area reduction portion, and the elastic member is located inside the depressed portion. According to this configuration, it is possible to dispose the second cross-sectional area reduction portion in an area covered with the elastic member. Therefore, it is possible to easily maintain the vibration transmission member in a clean state without allowing a piece of the body tissue, etc., to be accumulated in the second cross-sectional area reduction portion.

The vibration transmission member includes the bend portion 56 provided astride the distal end portion 46 and the intermediate portion 51; and the second cross-sectional area reduction portion is provided opposite to the first cross-sectional area reduction portion with respect to the plane P including the central axis C and the entire bend portion 56 (plane P passing along the central axis C and being approximately parallel to the bending direction of the bend portion 56). According to this configuration, it is possible to provide the second cross-sectional area reduction portion opposite to the first cross-sectional area reduction portion with respect to the plane P, and therefore possible to set off the abnormal vibration generated due to the first cross-sectional area reduction portion by using the second cross-sectional area reduction portion located opposite to the first cross-sectional area reduction portion.

The medical device includes the jaw 26 that faces the treatment face 52 and can be opened and closed relative to the treatment face 52. According to this configuration, it is possible to cause the vibration transmission member to perform ultrasonic vibration (resonate) while the body tissue is held between the jaw 26 and the vibration transmission member, and also possible to realize a favorable operation process without allowing the body tissue to be shifted during treatment.

The second cross-sectional area reduction portion is provided closer to the proximal direction C2 side than the treatment face 52. According to this configuration, it is possible to form the second cross-sectional area reduction portion apart from the treatment face 52, and possible to provide the treatment face 52 having a flat shape even if the second cross-sectional area reduction portion is provided. Thereby, it is possible to uniform the pressure applied to the body tissue when the vibration transmission member is brought into contact with the body tissue. Thereby, it is possible to uniform the performance of coagulating the body tissue and the performance of incising the body tissue on the treatment face 52.

A medical device system includes the medical device and the transducer described above. According to this configuration, the ultrasonic vibration generated in the transducer can be efficiently resonated on the medical device side, therefore saving power of the medical device system, reducing an amount of heat generated in the vibration transmission member, extending the life of the vibration transmission member, and reducing an amount of bubbles generated from the vibration transmission member at the time of the treatment.

The present invention is not limited by the above disclosure, but can be modified as appropriate in practice within the scope of the invention, the scope of which is by the claims.

## Claims

1. A vibration transmission member (15) configured to resonate upon transmission of ultrasonic vibration thereto from a transducer (16) that is configured to generate the ultrasonic vibration, the vibration transmission member (15) comprising:
a distal end portion (46);
a proximal end portion (47) provided closer to the transducer (16);
an intermediate portion (51) provided between the distal end portion (46) and the proximal end portion (47);
an inclined portion (54), which is provided to the distal end portion (46), and is inclined with respect to a central axis (C) of the vibration transmission member (15) in a state that the inclined portion (54) is closer to the central axis (C) as the inclined portion (54) is closer to a distal end of the distal end portion (46), and
a first cross-sectional area reduction portion (54), which makes a cross-sectional area of the distal end portion (46) concerning a plane intersecting with the central axis (C) of the vibration transmission member (15) smaller than a cross-sectional area of the intermediate portion (51) concerning a plane intersecting with the central axis (C) by the inclined portion (54),
whereby
the vibration transmission member (15) further includes:
a second cross-sectional area reduction portion (55) which is formed in the proximal end portion (47) so that an abnormal vibration due to the first cross-sectional area reduction portion (54) formed in the distal end portion is set off by an abnormal vibration due to the second cross-sectional area reduction portion (55), at which a node position of the ultrasonic vibration nearest to the distal end portion (46) is located while the vibration transmission member (15) is resonating, and which is provided opposite to the first cross-sectional area reduction portion (54) with respect to the central axis (C), the second cross-sectional area reduction portion (55) making a cross-sectional area concerning a plane intersecting with the central axis (C) of a half portion of the vibration transmission member (15) located opposite to the first cross-sectional area reduction portion (54) smaller than a cross-sectional area concerning a plane intersecting with the central axis (C) of a half portion of the vibration transmission member (15) located closer to the first cross-sectional area reduction portion (54), the second cross-sectional area reduction portion (55) having a shape different from that of the first cross-sectional area reduction portion (54) and asymmetrical to that of the first cross-sectional area reduction portion (54) with respect to the central axis (C).

2. The vibration transmission member (15) according to claim 1, wherein the second cross-sectional area reduction portion (55) is provided opposite to the first cross-sectional area reduction portion (54) at an angle of 180 degrees around the central axis (C).

3. The vibration transmission member (15) according to claim 1, wherein the second cross-sectional area reduction portion (55) forms a groove extending in a direction intersecting with the central axis (C).

4. The vibration transmission member (15) according to claim 1, wherein the second cross-sectional area reduction portion (55) forms a groove, and has an irregular cross-sectional shape when cut along a plane passing along the central axis (C).

5. The vibration transmission member (15) according to claim 1, wherein:
the vibration transmission member (15) includes a treatment face (52) that is provided opposite to the first cross-sectional area reduction portion (54) with respect to the central axis (C); and
a distance from a bottom portion of the groove formed by the second cross-sectional area reduction portion (55) to the central axis (C) is larger than a distance from the treatment face (52) to the central axis (C).

6. The vibration transmission member (15) according to claim 1,
wherein an annular depressed portion (62) is provided at a position overlapping with the second cross-sectional area reduction portion (55), and a part of an elastic member (33) is located inside the depressed portion.

7. The vibration transmission member (15) according to claim 1,
comprising a bend portion (56) that is provided astride the distal end portion (46) and the intermediate portion (51); and
the second cross-sectional area reduction portion (55) is provided opposite to the first cross-sectional area reduction portion (54) with respect to a plane that includes the central axis (C) and the bend portion (56).

8. The vibration transmission member (15) according to claim 1, comprising a treatment face (52) that is provided opposite to the first cross-sectional area reduction portion (54) with respect to the central axis (C),
wherein the second cross-sectional area reduction portion (55) is provided closer to a proximal direction side than the treatment face (52).

9. The vibration transmission member (15) according to claim 1, wherein the inclined portion (54) is provided to a back face (53) located opposite to a treatment face (52).

10. The vibration transmission member (15) according to claim 1, further comprising:
a treatment face (52); and
a back face (53) located opposite to the treatment face (52),
wherein the distal end portion (46) is bent toward a direction perpendicular to a thickness direction as the distal end portion (46) is closer to a distal direction side, the thickness direction being a direction from the treatment face (52) to the back face (53).

11. A medical device (12), comprising:
the vibration transmission member (15) of any of the preceding claims; and
a transducer (16) that is configured to generate ultrasonic vibration for resonating the vibration transmission member (15).

12. The medical device (12) according to claim 11, further comprising:
a cylindrical sheath-shaped section (34) that covers the vibration transmission member (15); and
an annular elastic member (33) that is interposed between the sheath-shaped section (34) and the vibration transmission member (15),
wherein an annular depressed portion (62) is provided to the vibration transmission member (15) at a position overlapping with the second cross-sectional area reduction portion (55), and a part of the elastic member (33) is located inside the depressed portion (62).

13. The medical device (12) according to claim 11, wherein:
the vibration transmission member (15) comprises a treatment face (52); and
the medical device (12) comprises a jaw (26) that faces the treatment face (52) and is openable and closable relative to the treatment face (52).

## Patentansprüche

1. Schwingungsübertragungselement (15), das so konfiguriert ist, dass es bei der Übertragung von Ultraschallschwingungen von einem Wandler (16), der so konfiguriert ist, dass er die Ultraschallschwingungen erzeugt, in Resonanz tritt, wobei das Schwingungsübertragungselement (15) umfasst:
einen distalen Endabschnitt (46);
einen proximalen Endabschnitt (47), der näher am Wandler (16) vorgesehen ist;
einen Zwischenabschnitt (51), der zwischen dem distalen Endabschnitt (46) und dem proximalen Endabschnitt (47) vorgesehen ist;
einen geneigten Abschnitt (54), der an dem distalen Endabschnitt (46) vorgesehen ist und in Bezug auf eine Mittelachse (C) des Schwingungsübertragungselements (15) in einem Zustand geneigt ist, dass der geneigte Abschnitt (54) näher an der Mittelachse (C) ist, wenn der geneigte Abschnitt (54) näher an einem distalen Ende des distalen Endabschnitts (46) ist, und
einen ersten Querschnittsflächenverringerungsabschnitt (54), der eine Querschnittsfläche des distalen Endabschnitts (46) bezüglich einer Ebene, die die Mittelachse (C) des Schwingungsübertragungselements (15) schneidet, kleiner macht als eine Querschnittsfläche des Zwischenabschnitts (51) bezüglich einer Ebene, die die Mittelachse (C) durch den geneigten Abschnitt (54) schneidet,
wobei das Schwingungsübertragungselement (15) ferner enthält:
einen zweiten Querschnittsflächenverringerungsabschnitt (55), der in dem proximalen Endabschnitt (47) ausgebildet ist, so dass eine anormale Schwingung aufgrund des ersten Querschnittsflächenverringerungsabschnitts (54), der in dem distalen Endabschnitt ausgebildet ist, durch eine anormale Schwingung aufgrund des zweiten Querschnittsflächenverringerungsabschnitts (55) ausgelöst wird, an dem sich eine dem distalen Endabschnitt (46) nächstgelegene Knotenposition der Ultraschallschwingung befindet, während das Schwingungsübertragungselement (15) in Resonanz ist, und der in Bezug auf die Mittelachse (C) gegenüber dem ersten Querschnittsflächenverringerungsabschnitt (54) vorgesehen ist, der zweite Querschnittsflächenverringerungsabschnitt (55) eine Querschnittsfläche, die eine Ebene betrifft, die die Mittelachse (C) eines halben Abschnitts des Schwingungsübertragungselements (15) schneidet, der gegenüber dem ersten Querschnittsflächenverringerungsabschnitt (54) angeordnet ist, kleiner macht als eine Querschnittsfläche, die eine Ebene betrifft, die die Mittelachse (C) eines halben Abschnitts des Schwingungsübertragungselements (15) schneidet, der näher an dem ersten Querschnittsflächenverringerungsabschnitt (54) angeordnet ist, der zweite Querschnittsflächenverringerungsabschnitt (55) eine Form hat, die sich von der des ersten Querschnittsflächenverringerungsabschnitts (54) unterscheidet und asymmetrisch zu der des ersten Querschnittsflächenverringerungsabschnitts (54) in Bezug auf die Mittelachse (C) ist.

2. Schwingungsübertragungselement (15) gemäß Anspruch 1, wobei der zweite Querschnittsflächenverringerungsabschnitt (55) gegenüber dem ersten Querschnittsflächenverringerungsabschnitt (54) in einem Winkel von 180 Grad um die Mittelachse (C) vorgesehen ist.

3. Schwingungsübertragungselement (15) gemäß Anspruch 1, wobei der zweite Querschnittsflächenverringerungsabschnitt (55) eine Nut bildet, die sich in einer Richtung erstreckt, die die Mittelachse (C) schneidet.

4. Schwingungsübertragungselement (15) gemäß Anspruch 1, wobei der zweite Querschnittsflächenverringerungsabschnitt (55) eine Nut bildet und eine unregelmäßige Querschnittsform aufweist, wenn er entlang einer Ebene geschnitten wird, die durch die Mittelachse (C) verläuft.

5. Schwingungsübertragungselement (15) gemäß Anspruch 1, wobei:
das Schwingungsübertragungselement (15) eine Behandlungsfläche (52) enthält, die gegenüber dem ersten Querschnittsflächenverringerungsabschnitt (54) in Bezug auf die Mittelachse (C) vorgesehen ist; und
ein Abstand von einem Bodenabschnitt der durch den zweiten Querschnittsflächenverringerungsabschnitt (55) gebildeten Nut zu der Mittelachse (C) größer ist als ein Abstand von der Behandlungsfläche (52) zu der Mittelachse (C).

6. Schwingungsübertragungselement (15) gemäß Anspruch 1,
wobei ein ringförmiger vertiefter Abschnitt (62) an einer Position vorgesehen ist, die mit dem zweiten Querschnittsflächenverringerungsabschnitt (55) überlappt, und ein Teil eines elastischen Elements (33) innerhalb des vertieften Abschnitts angeordnet ist.

7. Schwingungsübertragungselement (15) gemäß Anspruch 1,
umfassend einen gebogenen Abschnitt (56), der rittlings auf dem distalen Endabschnitt (46) und dem Zwischenabschnitt (51) angeordnet ist; und
der zweite Querschnittsflächenverringerungsabschnitt (55) gegenüber dem ersten Querschnittsflächenverringerungsabschnitt (54) in Bezug auf eine Ebene vorgesehen ist, die die Mittelachse (C) und den Biegeabschnitt (56) enthält.

8. Schwingungsübertragungselement (15) gemäß Anspruch 1 umfassend eine Behandlungsfläche (52), die in Bezug auf die Mittelachse (C) gegenüber dem ersten Abschnitt (54) zur Verringerung der Querschnittsfläche vorgesehen ist,
wobei der zweite Querschnittsflächenverringerungsabschnitt (55) näher an einer Seite in proximaler Richtung als die Behandlungsfläche (52) vorgesehen ist.

9. Schwingungsübertragungselement (15) gemäß Anspruch 1, wobei der geneigte Abschnitt (54) an einer Rückfläche (53) vorgesehen ist, die einer Behandlungsfläche (52) gegenüberliegt.

10. Schwingungsübertragungselement (15) gemäß Anspruch 1, ferner umfassend:
eine Behandlungsfläche (52); und
eine Rückseite (53), die der Behandlungsseite (52) gegenüberliegt,
wobei der distale Endabschnitt (46) in eine Richtung senkrecht zu einer Dickenrichtung gebogen ist, wenn der distale Endabschnitt (46) näher an einer distalen Richtungsseite ist, wobei die Dickenrichtung eine Richtung von der Behandlungsfläche (52) zu der Rückfläche (53) ist.

11. Medizinische Vorrichtung (12), umfassend:
das Schwingungsübertragungselement (15) nach einem der vorhergehenden Ansprüche; und
einen Wandler (16), der so konfiguriert ist, dass er Ultraschallschwingungen erzeugt, um das Schwingungsübertragungselement (15) in Resonanz zu bringen.

12. Medizinische Vorrichtung (12) nach Anspruch 11, ferner umfassend:
einen zylindrischen, mantelförmigen Abschnitt (34), der das Schwingungsübertragungselement (15) abdeckt; und
ein ringförmiges elastisches Element (33), das zwischen dem hülsenförmigen Abschnitt (34) und dem Schwingungsübertragungselement (15) angeordnet ist,
wobei ein ringförmiger vertiefter Abschnitt (62) an dem Schwingungsübertragungselement (15) an einer Position vorgesehen ist, die mit dem zweiten Querschnittsflächenverringerungsabschnitt (55) überlappt, und ein Teil des elastischen Elements (33) innerhalb des vertieften Abschnitts (62) angeordnet ist.

13. Medizinische Vorrichtung (12) nach Anspruch 11, wobei:
das Schwingungsübertragungselement (15) eine Behandlungsfläche (52) umfasst; und
die medizinische Vorrichtung (12) eine Backe (26) umfasst, die der Behandlungsfläche (52) zugewandt ist und relativ zur Behandlungsfläche (52) geöffnet und geschlossen werden kann.

## Revendications

1. Élément de transmission de vibrations (15) conçu pour entrer en résonance lors de la transmission de vibrations provenant d'un transducteur (16) qui est conçu pour générer la vibration ultrasonore, l'élément de transmission de vibrations (15) comprenant:
une partie d'extrémité distale (46);
une partie d'extrémité proximale (47) plus proche du transducteur (16);
une partie intermédiaire (51) disposée entre la partie d'extrémité distale (46) et la partie d'extrémité proximale (47);
une partie inclinée (54), qui est disposée sur la partie d'extrémité distale (46), et est inclinée par rapport à un axe central (C) de l'élément de transmission de vibrations (15) lorsque la partie inclinée (54) est plus proche d'une extrémité distale de la partie d'extrémité distale (46), et
une première partie de réduction de section transversale (54), qui rend la section transversale de la partie d'extrémité distale (46) concernant un plan coupant l'axe central (C) de l'élément de transmission de vibrations (15) plus petite que la section transversale de la partie intermédiaire (51) concernant un plan coupant l'axe central (C) par la partie inclinée (54),
l'élément de transmission de vibrations (15) comprenant en outre:
une deuxième partie de réduction de section transversale (55) qui est formée dans la partie d'extrémité proximale (47) de sorte qu'une vibration anormale due à la première partie de réduction de la section transversale (54) formée dans la partie d'extrémité distale est compensée par une vibration anormale due à la deuxième partie de réduction de la section transversale (55), à laquelle se trouve une position de noeud de la vibration ultrasonore la plus proche de la partie d'extrémité distale (46) lorsque l'élément de transmission de vibrations (15) résonne, et qui est située en regard de la première partie de réduction de section transversale (54) par rapport à l'axe central (C), la seconde partie de réduction de section transversale (55) rend une section transversale concernant un plan coupant l'axe central (C) d'une demi-portion de l'élément de transmission de vibrations (15) située en regard de la première partie de réduction de section transversale (54) plus petite qu'une section transversale concernant un plan coupant l'axe central (C) d'une demi-portion de l'élément de transmission de vibrations (15) située plus près de la première partie de réduction de section transversale (54), la seconde partie de réduction de section transversale (55) ayant une forme différente de celle de la première partie de réduction de section transversale (54) et asymétrique par rapport à celle de la première partie de réduction de section transversale (54) par rapport à l'axe central (C).

2. Élément de transmission de vibrations (15) selon la revendication 1, dans lequel la seconde partie de réduction de section transversale (55) est disposée en regard de la première partie de réduction de section transversale (54) à un angle de 180 degrés autour de l'axe central (C).

3. Élément de transmission de vibrations (15) selon la revendication 1, dans lequel la seconde partie de réduction de section transversale (55) forme une rainure partant dans une direction coupant l'axe central (C).

4. Élément de transmission de vibrations (15) selon la revendication 1, dans lequel la seconde partie de réduction de section transversale (55) forme une rainure, et a une forme transversale irrégulière lorsqu'elle est coupée le long d'un plan passant le long de l'axe central (C).

5. Élément de transmission de vibrations (15) selon la revendication 1, dans lequel:
l'élément de transmission de vibrations (15) comprend une face de traitement (52) qui se trouve en regard de la première partie de réduction de section transversale (54) par rapport à l'axe central (C); et
une distance d'une partie de la rainure formée par la seconde partie de réduction de section transversale (55) à l'axe central (C) est supérieure à une distance de la face de traitement (52) à l'axe central (C).

6. Élément de transmission de vibrations (15) selon la revendication 1,
dans lequel une partie annulaire en retrait (62) est positionnée dans une position chevauchant la seconde partie de réduction de section transversale (55), et une partie d'un élément élastique (33) est située à l'intérieur de la partie en retrait.

7. Élément de transmission de vibrations (15) selon la revendication 1,
comprenant une partie pliée (56) qui se trouve à cheval sur la partie d'extrémité distale (46) et la partie intermédiaire (51); et
la seconde partie de réduction de section transversale (55) est disposée en regard de la première partie de réduction de section transversale (54) par rapport à un plan qui comprend l'axe central (C) et la partie pliée (56).

8. Élément de transmission de vibrations (15) selon la revendication 1, comprenant une face de traitement (52) qui est disposée en regard de la première partie de réduction de section transversale (54) par rapport à l'axe central (C),
dans lequel la seconde partie de réduction de section transversale (55) est plus proche d'un côté de direction proximale que la face de traitement (52).

9. Élément de transmission de vibrations (15) selon la revendication 1, dans lequel la partie inclinée (54) est disposée sur une face arrière (53) se trouvant en regard d'une face de traitement (52).

10. Élément de transmission de vibrations (15) selon la revendication 1, comprenant en outre:
une face de traitement (52); et
une face arrière (53) en regard de la face de traitement (52),
dans lequel la partie d'extrémité distale (46) est pliée dans une direction perpendiculaire à une direction d'épaisseur à mesure que la partie d'extrémité distale (46) est plus proche d'un côté de direction distale, la direction de l'épaisseur étant une direction allant de la face de traitement (52) à la face arrière (53).

11. Dispositif médical (12) comprenant:
l'élément de transmission de vibrations (15) selon l'une quelconque des revendications précédentes; et
un transducteur (16) qui est conçu pour générer une vibration ultrasonore qui entre en résonance avec l'élément de transmission de vibrations (15).

12. Dispositif médical selon la revendication 11, comprenant en outre:
une section cylindrique en forme de gaine (34) qui recouvre l'élément de transmission de vibrations (15); et
un élément élastique annulaire (33) qui est intercalé entre la section en forme de gaine (34) et l'élément de transmission de vibrations (15),
dans lequel une partie annulaire en retrait (62) est positionnée sur l'élément de transmission de vibrations (15) dans une position chevauchant la seconde partie de réduction de section transversale (55), et une partie de l'élément élastique (33) est située à l'intérieur de la partie en retrait (62).

13. Dispositif médical (12) selon la revendication 11, dans lequel:
l'élément de transmission de vibrations (15) comprenant une face de traitement (52); et
le dispositif médical (12) comprend une mâchoire (36) qui fait face à la face de traitement (52) et qui peut s'ouvrir et se fermer par rapport à la face de traitement (52).
